# EUROPEAN PATENT APPLICATION

(11) **EP 1 637 867 A1**
(43) Date of publication of application: **22.03.2006**
(21) Application number: 04727987.2
(22) Date of filing: 16.04.2004
(51) Int. Cl.: G01N 13/16, G01N 33/53, G01N 37/00, C12N 15/00, C12Q 1/68

(54) **MOLECULE DETECTING METHOD, MOLECULE COUNTING METHOD, MOLECULE LOCALIZATION DETECTING METHOD, MOLECULE DETECTION DEVICE USED FOR THEM**

(30) Priority: 18.04.2003 JP 2003114836; 26.12.2003 JP 2003433969
(71) Applicant: Hitachi Chemical Company, Ltd., Tokyo 163-0449 (JP)
(72) Inventor: KOBAYASHI, Teruyuki c/o Hitachi Chemical Co., Ltd, Ibaraki 300-4247 (JP); NAKAYAMA, Noriyuki c/o Hitachi Chemical Co., Ltd, Ibaraki 300-4247 (JP); TAMURA, Tsuruki c/o Hitachi Chemical Co., Ltd, Ibaraki 300-4247 (JP); KIM, Munsok c/o Hitachi Chemical Co., Ltd, Tokyo 163-0449 (JP)
(74) Representative: HOFFMANN EITLE
(86) International application number: PCT/JP2004/005463
(87) International publication number: WO 2004/092712

(57) **Abstract**

The present invention relates to a molecular detection method in which a chain molecule immobilized on a substrate is visualized and identified by probing with a scanning probe microscope in solution. Furthermore, the present invention relates to a system for detecting a chain molecule immobilized on a substrate, the system being equipped with a jig for holing the substrate, a container housing the substrate and a solution, a probe, a probe detector, a drive mechanism for scanning the substrate or the probe in three dimensions, and a drive control circuit for controlling the drive mechanism. In accordance with the present invention, a chain molecule immobilized on a substrate can be detected clearly, which has been difficult conventionally.

## Description

### Technical Field

The present invention relates to a molecular detection method, counting method, and localization detection method. More particularly, it relates to a method that enables a chain molecule to be visualized and identified by probing a molecule by means of a probe of a scanning probe microscope.

Furthermore, the present invention relates to a molecular detection system for detecting a chain molecule immobilized on a substrate. More particularly, it relates to a molecular detection system that, by probing a molecule by means of a probe, enables a chain molecule standing upright to be visualized, the number of chain molecules per unit area to be counted, molecular localization information to be obtained, etc.

Moreover, the present invention relates to a production process for a substrate with a chain molecule immobilized thereon, the production process employing the above-mentioned method or system.

### Background Art

As methods for immobilizing DNA on a substrate, there are a method in which DNA is directly synthesized on a substrate so as to immobilize it, and a method in which DNA that has been synthesized separately is immobilized on a substrate. The technique employed in the former method is a photolithographic technique, and this technique produces a DNA chip. The latter method employs a mechanical microspotting technique, and this technique produces a DNA microarray.

In the above-mentioned DNA chip or DNA microarray, if the DNA immobilized on the substrate is localized, the reliability of an analytical result such as gene expression information obtained using the chip or microarray is degraded. That is, unless the DNA is uniformly and distributedly (nonlocalized) immobilized on an intended section on the substrate, qualitative and quantitative analytical performance cannot be exhibited. Conventionally, there is no technique for examining, at the molecular level, whether or not single strand DNA is uniformly immobilized on a specified area on the substrate, and since the DNA chip and DNA microarray are very expensive, there has been a desire for the development of such a testing technique.

DNA immobilized on the DNA chip or DNA microarray can be detected by subjecting the immobilized DNA to hybridization with a fluorescently labeled complementary DNA and measuring the fluorescence intensity, but this method cannot give any information about molecular localization.

The above-mentioned problem with the DNA chip and DNA microarray can also be seen in a microtiter plate used for enzyme linked immunoassay (ELISA) or a protein chip. That is, by staining a protein molecule immobilized on a substrate with a dye, etc. having an affinity for the protein molecule and measuring the absorbance, binding a fluorescently labeled protein to the protein molecule and measuring the fluorescence intensity, etc., only an average value for the protein concentration per unit area can be obtained, and molecular localization information cannot be obtained.

With regard to means for obtaining information on whether or not immobilized molecules are nonlocalized or localized on a substrate, there is observation using an electron microscope, etc. However, since this observation is carried out under vacuum, in the case of biopolymers the structure thereof will be destroyed and observation is not possible, or they stick to the substrate, thus making it impossible to distinguish them from the substrate.

In addition to the above, a technique of imaging the surface of a substrate by means of UV spectroscopy, IR spectroscopy, etc. has been employed in recent years, but since due to the structure of the equipment the field of vision of the measurement is wide, it is difficult to obtain localization information at the molecular level.

On the other hand, in order to visualize a molecule and obtain information about its shape, observation using a scanning probe microscope is known. With regard to a nucleic acid, the helical structure of a double strand nucleic acid has been confirmed by observation using a scanning probe microscope (ref. e.g. T. P. Beebe, Jr., T. E. Wilson, D. F. Ogletree, J. E. Katz, R. Balhorn, M. B. Salmeron, and W. J. Siekhaus, Science, 243, 370 (1989), or R. J. Driscoll, M. G. Youngquist, and J. D. Baldeschwieler, Nature, 346, 294 (1990)). It also becomes possible to distinguish between single strand DNA and double strand DNA by atomic force measurement using an atomic force microscope (ref. e.g. J. Wang and A. J. Bard, Anal. Chem., 73, 2207 (2001)). However, in these techniques, since the nucleic acid is made to adsorb parallel to the substrate, the degree of freedom as a molecule is restricted, and it must be said that observation is carried out in an inactive state, which is not desirable for a subsequent reaction.

From the viewpoint of the measurement environment, since there is a difference in environment between measurement in air and in solution, molecules stick to the substrate in air, but move around freely in solution. When molecules are measured in air, adjacent molecules are intertwined with each other and stick to the substrate, and individual molecules cannot be identified.

In the above-mentioned DNA chip, DNA microarray, etc., the chain molecule is immobilized on the substrate while maintaining an active state. That is, by making the molecule stand upright on the substrate, the degree of freedom of the molecule is increased, and the reaction site is made more open, thus improving the reactivity. Therefore, the above-mentioned observation method in which a nucleic acid is made to adsorb parallel to the substrate is not suitable for observation of the DNA chip, DNA microarray, etc.

Furthermore, with regard to a system for analyzing DNA immobilized on the surface of a substrate, a system employing a scanning probe microscope is known, and this system is employed in a method for determining the base sequence of one DNA chain (ref. e.g. Japanese Patent Application Laid-open Nos. 6-289017 and 2001-124687).

Moreover, there is a known method in which an immunoreaction of a material to be detected is counted not by fluorescence but by a scanning probe microscope (ref. e.g. Japanese Patent Publication No. 3386883). This method is a method in which a single molecule membrane base having good uniformity is formed and molecules of a single type are uniformly aligned; the conditions of the method are thus limited, it cannot be said that the environment of the DNA chip or ELISA is reproduced, and this method cannot be used for confirming localization or nonlocalization of molecules in order to examine whether or not molecules on the DNA chip or ELISA are uniformly immobilized.

Under such circumstances, there is a desire for a molecular detection system that enables, in a substrate such as a DNA chip or a DNA microarray on which a large number of chain molecules are immobilized, the molecules to be visualized and counted while maintaining the activity of the chain molecules and, furthermore, information about localization of the molecules to be obtained.

It is an object of the present invention to provide a molecular detection method, counting method, and localization detection method that enable chain molecules immobilized on a substrate to be clearly identified while maintaining an active state thereof. It is also an object of the present invention to provide an accurate detection method, counting method, and localization detection method for molecules on a substrate, the methods involving making an upright single strand molecule rigid so that it can withstand a scanning probe microscope, and carrying out measurement in solution so that the upright molecule does not stick to the substrate.

It is another object of the present invention to provide a system for detecting a chain molecule immobilized on a substrate while maintaining an active state. It is yet another object of the present invention to provide a molecular detection system that enables a chain molecule immobilized on a substrate to be clearly visualized while maintaining an active state, the number of chain molecules to be counted, information about localization of chain molecules to be obtained, etc.

It is yet another object of the present invention to provide a production process for a substrate with a chain molecule immobilized thereon, the production process employing the above-mentioned method or the above-mentioned system.

### Disclosure of Invention

The present invention relates to a molecular detection method comprising visualizing and identifying a chain molecule immobilized on a substrate by probing with a scanning probe microscope in solution.

Furthermore, the present invention also relates to a molecular counting method wherein the number of detected chain molecules per unit area is counted by the above-mentioned method.

Moreover, the present invention also relates to a molecular localization detection method wherein the number of detected chain molecules per unit area is counted by the above-mentioned method, thus giving molecular localization information.

Furthermore, the present invention also relates to a molecular detection system for detecting a chain molecule immobilized on a substrate, the system comprising a jig for holding the substrate, a container housing the substrate and a solution, a probe, a probe detector, a drive mechanism for scanning the substrate or the probe in three dimensions, and a drive control circuit for controlling the drive mechanism. The molecular detection system preferably further comprises a device which visualizes the chain molecule. Furthermore, the molecular detection system preferably further comprises a device which counts the chain molecules. Moreover, the molecular detection system preferably further comprises a device which provides information about localization of the chain molecules. Furthermore, the molecular detection system further comprises a device which discriminates between substrates with chain molecules immobilized thereon.

Moreover, the present invention also relates to a production process for a substrate with a chain molecule immobilized thereon, the production process including the above-mentioned method, or a production process for a substrate with a chain molecule immobilized thereon, the production process employing the above-mentioned system.

In the present invention, the chain molecule immobilized on the substrate is preferably a single strand molecule uprightly disposed on the substrate, and the uprightly disposed single strand molecule is more preferably a nucleic acid, a peptide nucleic acid, a peptide, a glycopeptide, a protein, a glycoprotein, a polysaccharide, a synthetic polymer, or an analog thereof.

Furthermore, in the present invention, the chain molecule immobilized on the substrate is a multiple strand molecule comprising an uprightly disposed single strand molecule and at least one chain molecule that can bind to the single strand molecule, and the multiple strand molecule is more preferably a complex of one or more types of molecules selected from a nucleic acid, a peptide nucleic acid, a peptide, a glycopeptide, a protein, a glycoprotein, a polysaccharide, a synthetic polymer, or an analog thereof.

The disclosures of the present invention relate to subject matter described in Japanese Patent Application No. 2003-114836, filed on April 18th, 2003, and Japanese Patent Application No. 2003-433969, filed on December 26th, 2003, and the contents of the disclosures therein are incorporated herein by reference.

### Brief Description of Drawings

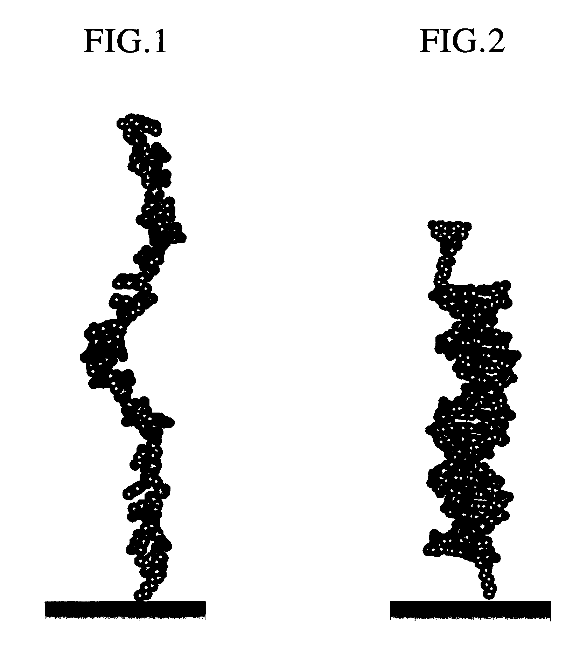

FIG. 1 is a space filling model of a single strand DNA immobilized on a substrate.
FIG. 2 is a space filling model of a double helix DNA formed from a single strand DNA immobilized on a substrate and a DNA with bases that form complementary pairs therewith.
FIG. 3 (a) is an image of a plastic substrate observed with an atomic force microscope and FIG 3 (b) is a line profile at a given point.
FIG. 4 (a) is an image of a plastic substrate having dT₂₀ immobilized thereon observed with an atomic force microscope, and FIG. 4 (b) is a line profile at a given point.
FIG. 5 (a) is an image of the sample shown in FIG. 4 to which dA₂₀-FAM has been added so as to form a double helix observed with an atomic force microscope, and FIG. 5 (b) is a line profile at a given point.
FIG. 6 is an image of the sample shown in FIG. 5 observed in air with an atomic force microscope.
FIG. 7 (a) is a schematic diagram of a single strand DNA immobilized on a substrate when observed with an atomic force microscope, and FIG. 7 (b) is a schematic diagram of a double strand DNA having the same number of bases when observed with an atomic force microscope. In FIG. 7, 31 denotes a substrate, 32 denotes a dT₂₀ single strand nucleic acid, 33 denotes double strand dT₂₀ and dA₂₀-FAM nucleic acids, 34 denotes the particle shape observed for single strand DNA, and 35 denotes the particle shape observed for double strand DNA.
FIG. 8 (a) is a particle analysis diagram of the substrate shown in FIG. 3, and FIG. 8 (b) is a histogram in which the abscissa is the proportion of particles (number of particles) and the ordinate is the particle height.
FIG. 9 (a) is a particle analysis diagram of the substrate with dT₂₀ immobilized thereon shown in FIG. 4, and FIG. 9 (b) is a histogram in which the abscissa is the proportion of particles (number of particles) and the ordinate is the particle height.
FIG. 10 (a) is a particle analysis diagram of the substrate shown in FIG. 5, on which the double helix has been formed, and FIG. 10 (b) is a histogram in which the abscissa is the proportion of particles (number of particles) and the ordinate is the particle height.
FIG. 11 is a diagram for explaining the principle of a molecular detection system (AFM) of one embodiment of the present invention.
FIG. 12 is a diagram for explaining the principle of a molecular detection system (STM) of one embodiment of the present invention.
FIG. 13 is a flowchart showing the operation of a molecular detection system having a visualization device of the present invention.
FIG. 14 is a flowchart showing the operation of a molecular detection system having a counting device of the present invention.
FIG. 15 is a flowchart showing the operation of a molecular detection system having a localization information providing device of the present invention.
FIG. 16 is a flowchart showing the operation of a substrate discrimination device of the present invention.
FIG. 17 is a UV-irradiated polystyrene substrate visualized using the molecular detection system of the present invention.
FIG. 18 is a substrate with a chain molecule immobilized thereon visualized using the molecular detection system of the present invention.
FIG. 19 is a substrate with a double helix immobilized thereon visualized using the molecular detection system of the present invention.
FIG. 20 is a diagram showing particles counted on a UV-irradiated polystyrene substrate after removing noise in the height direction and the plane direction.
FIG. 21 is a diagram showing particles counted on a substrate with dT₂₀ immobilized thereon after removing noise in the height direction and the plane direction.
FIG. 22 is a diagram showing particles counted on a substrate with a double helix immobilized thereon after removing noise in the height direction and the plane direction.
FIG. 23 is a line profile obtained by bisecting a topographic image of a given particle at a sectional plane passing through the vertex of the particle.
FIG. 24 is an image of a mica substrate with lysozyme immobilized thereon observed with an atomic force microscope.
FIG. 25 is an image of the sample shown in FIG. 24 to which an anti-lysozyme antibody has been added so as to form an antigen-antibody complex observed with an atomic force microscope.
FIG. 26 is a particle analysis diagram of the substrate with lysozyme immobilized thereon shown in FIG. 24.
FIG. 27 is a particle analysis diagram of the lysozyme/anti-lysozyme antibody complex shown in FIG. 25.

### Best Mode for Carrying Out the Invention

The present invention is explained in detail below.

In the present invention, a chain molecule, which is a detection target, generally has a length (height) that is greater than the roughness of a substrate, and is usually either a single strand molecule or a multiple strand molecule. A plurality of chain molecules are usually immobilized on the substrate, and the plurality of chain molecules may be identical to or different from each other. That is, the plurality of chain molecules may have the same length (height) or different lengths (heights). Furthermore, the chain molecules as the detection target may be uniformly distributedly (nonlocalized) immobilized or may be nonuniformly (localized) immobilized. In addition, the chain molecules as the detection target may have restricted orientation or unrestricted orientation.

Chain molecules observed using a molecular detection system of the present invention are preferably chain molecules immobilized on various types of arrays, chips, or microtiter plates.

### <Arrangement of single strand molecule>

In the present invention, the single strand molecule is generally a nucleic acid such as a deoxyribonucleic acid (DNA), a ribonucleic acid (RNA), an artificial nucleic acid having adenine, thymine, cytosine, guanine, uracil, or hypoxanthine, or a nucleic acid derivative, or a peptide nucleic acid (PNA). Furthermore, one having a complementary or specific molecule such as a peptide, a glycopeptide, a protein, a glycoprotein, a polysaccharide, a synthetic polymer (e.g. polymethacrylic acid, polyacrylic acid, polyvinylimidazole, polystyrenesulfonic acid, polyallylamine, polyacrylamide, polythiopheneacetic acid, or polypyridylacetylene), or an analog thereof is preferable since the above-mentioned multiple strand molecule may be formed. It is also possible to use a synthetic polymer such as polyphenol, polyester, polyethylene glycol, polyamide acid, polyvinylpyrrolidone, or polyvinylalcohol.

With regard to the single strand molecule, for example, one having in part a branched structure or a network structure may be used. The single strand molecule having in part a branched structure or a network structure referred to in the present invention includes not only a molecule having a branched structure or a network structure by virtue of a covalent bond such as a proteoglycan or a synthetic polymer having a side chain, but also a molecule having a branched structure or a network structure by virtue of hydrogen bonding, ionic bonding, hydrophobic binding, etc. of a peptide chain within the molecule, such as a protein higher order structure.

The single strand molecule that is to be detected by the molecular detection system of the present invention is not limited to the above, and any molecule that can be detected may become a detection target.

The above-mentioned single strand molecule is normally disposed on (immobilized on) a substrate in an upright state. Disposing on (immobilizing on) the substrate referred to in the present invention means immobilizing by means of indirect bonding via a linker, or any physical adsorption or chemical bonding including electrostatic binding, hydrophobic binding, ionic bonding, and hydrogen bonding.

The substrate referred to above generally includes a plate, a bead, a well, a membrane, and a film, which are made of a material such as plastic, glass, or metal, and is usually a plate.

The method for immobilizing a single strand molecule on a substrate is not particularly limited, and it is possible to employ a method in which a solution containing the single strand molecule is dropped on a substrate having a functional group, etc. incorporated thereinto as necessary and incubated, etc. In the present invention, it is therefore possible to use a substrate that does not restrict the orientation of the chain molecules, that is, a substrate that does not control the direction in which the molecules are arranged. It is preferable to employ the substrate thus obtained since it does not require any complicated procedure for immobilizing a chain molecule and it may be obtained by any method. It is of course possible to use in the present invention a substrate in which the arrangement direction is controlled, that is, the orientation of molecules is restricted. As a solution in this case, an aqueous solvent containing a salt such as sodium chloride, potassium chloride, ammonium chloride, sodium acetate, potassium acetate, ammonium acetate, or sodium phosphate, or a buffer solution, etc. may be used.

Examples of the substrate on which a single strand molecule has been immobilized include various types of arrays and chips such as a DNA chip, a DNA microarray, a protein array (protein chip), and a peptide array.

### <Formation of multiple strand molecule>

In the present invention, the chain molecule immobilized on the substrate may be a multiple strand molecule comprising an uprightly disposed single strand molecule and at least one chain molecule that can bind to the single strand molecule.

The multiple strand molecule referred to in the present invention is preferably a complex of one or more types of molecules selected from a nucleic acid, a peptide nucleic acid, a peptide, a glycopeptide, a protein, a glycoprotein, a polysaccharide, a synthetic polymer, and an analog thereof. The multiple strand molecule usually denotes a complex formed between the above-mentioned single strand molecule and a chain molecule that can be bonded therewith via various types of physical adsorption or chemical bonding including electrostatic binding, hydrophobic binding, ionic bonding, and hydrogen bonding between the molecules.

In the present invention, the binding between the single strand molecule and the chain molecule that can bind thereto is preferably complementary or specific binding. Therefore, examples of the multiple strand molecule referred to in the present invention include a double strand DNA, a double strand RNA, a double strand PNA, a hybrid of DNA and RNA, a hybrid of DNA and PNA, a hybrid of RNA and PNA, a hybrid of an artificial nucleic acid having adenine, thymine, cytosine, guanine, uracil, or hypoxanthine or a nucleic acid derivative with DNA, RNA, or PNA, and a complex such as a triple strand nucleic acid in which nucleic acids or peptide nucleic acids, which are chain molecules, are complementarily bound. Furthermore, the multiple strand molecule referred to in the present invention is not limited to the above, but includes all complexes in which chain molecules such as a peptide, a glycopeptide, a protein, a glycoprotein, a polysaccharide, or a synthetic polymer interact and bind to each other. Therefore, the multiple strand molecule referred to in the present invention includes an antibody comprising four polypeptide chains and an enzyme having a subunit structure, and further includes complexes such as an antigen-antibody complex, an enzyme-substrate complex, and an avidin-biotin complex, in which chain molecules are specifically bound.

Examples of the substrate having the multiple strand molecule immobilized thereon include a microtiter plate, an antibody array protein interaction array, and an enzyme array.

In the present invention, when the detection target is a single strand molecule uprightly disposed relative to the substrate, since in particular a single strand molecule having no branched structure or network structure bends on its own, if there are adjacent molecules they overlap each other, and it might become difficult to recognize them as individual molecules. It is therefore preferable to add one or more types of single strand molecule that can be bound to the arranged single strand molecule, thus forming a multiple strand molecule. When the chain molecule as the detection target is a short (low) molecule, it might be difficult to recognize it using the molecular detection system of the present invention. In this case also, it is preferable to add one or more types of chain molecule that can be bound to the arranged chain molecule, thus forming a long (high) molecule. The chain molecule that is bound for detection may be dissociated after carrying out detection using the molecular detection system.

For example, in the case of DNA, by adding a single strand DNA having a complementary base sequence, a double strand DNA is formed. By so doing, the molecular structure becomes rigid, resistance to contact with a probe can be introduced, and detection sensitivity improves, which are preferable. By scanning with the probe to give a profile, a multiple strand molecule is detected.

The conditions under which the multiple strand molecule is formed are not particularly limited, and it may be carried out in accordance with a standard method. For example, formation of a double strand DNA, that is, hybridization, is carried out in an aqueous solvent containing a salt such as sodium chloride, potassium chloride, ammonium chloride, sodium acetate, potassium acetate, ammonium acetate, or sodium phosphate, or a buffer solution. Formation of multiple strands containing a protein molecule, for example, binding of an antibody to a substrate on which an antigen is adsorbed (antigen-antibody reaction), is carried out in an aqueous solvent containing a salt such as sodium chloride, potassium chloride, ammonium chloride, sodium acetate, potassium acetate, ammonium acetate, or sodium phosphate, or a buffer solution.

### <Solution>

There is a difference between the environment in air and that in solution; in air, adjacent molecules are intertwined with each other and stick to a substrate, but in solution molecules move around freely.

In the present invention, by carrying out observation by means of a scanning probe microscope in solution, an image of the upright molecule on the substrate can be obtained. Since a single strand molecule tends to bend easily, and a double strand molecule is rigid and tends not to bend easily, the two can be distinguished by height, particularly when a multiple strand molecule is detected. A multiple strand molecule is resistant to the influence of an obstacle in the horizontal direction during probing.

The solution used in the detection method of the present invention is not particularly limited as long as the detection target can exist stably, and it is preferable to use an aqueous solution containing a salt such as sodium chloride, potassium chloride, ammonium chloride, sodium acetate, potassium acetate, or ammonium acetate, or a buffer solution, which are used when forming the above-mentioned multiple strand molecule.

By estimating the number of chain molecules having a height that is equal to or greater than a certain threshold value in the above-mentioned method, information about the localization of molecules can be obtained. By counting the number of chain molecules probed per unit area, the number of molecules can be counted.

### <Scanning probe microscope>

A scanning probe microscope used in the method of the present invention is a general term for scanning probe microscopes than enable observation at the atomic level, and includes a scanning tunneling microscope (STM) and an atomic force microscope (AFM).

AFM can be roughly divided into repulsive mode, attractive mode, tapping mode ('tapping mode' is a registered trademark of Digital Instruments, Inc., Santa Barbara, California, USA), etc. Research in this field is still in progress, and new microscopes are being developed. The scanning probe microscope referred to in the present invention is not limited to those currently known and includes microscopes that will be developed in the future as long as they have a resolution at the atomic level.

The probing method itself may be a standard method that is used with the above-mentioned microscopes, and it is preferable to carry out AFM observation in solution.

In the molecular detection method of the present invention, it is possible to use any of the scanning probe microscopes described above, and it is particularly preferable to use the molecular detection system, which is another invention of the present invention.

### <Molecular detection system>

The molecular detection system of the present invention is a system for detecting a chain molecule immobilized on a substrate, and includes a jig for holding the substrate, a container housing the substrate and a solution, a probe, a drive mechanism for scanning the substrate or the probe in three dimensions, and a drive control circuit for controlling the drive mechanism. As such a molecular detection system, a part or the entirety of the mechanism of a scanning probe microscope (SPM) such as an atomic force microscope (AFM) or a scanning tunneling microscope (STM) can be used.

The atomic force microscope is to observe a profile of the surface of a detection target by utilizing an atomic force acting between the detection target and a probe, that is, by measuring the amount of flexing of the probe caused by the atomic force. The amount of flexing is preferably measured by a method utilizing reflection of laser light, and it is therefore preferable for the molecular detection system of the present invention to have as a probe detector a laser light source and a laser light detector.

Atomic force microscopes can be roughly divided, in terms of differences in operation of the probe, into repulsive mode, attractive mode, tapping mode ('tapping mode' is a registered trademark of Digital Instruments, Inc., Santa Barbara, California, USA), etc. In the present invention, it is preferable to use a tapping mode atomic force microscope from the viewpoint of damage to the substrate having a chain molecule immobilized thereon being smaller than that from the repulsive mode and the amount of flexing of the probe being detected more easily than in the attractive mode.

The scanning tunneling microscope observes a profile of the surface of a detection target by measuring a tunnel current passing between the detection target and a probe. It is therefore preferable for the molecular detection system of the present invention to have as a probe detector a voltage generator and a tunnel current detector.

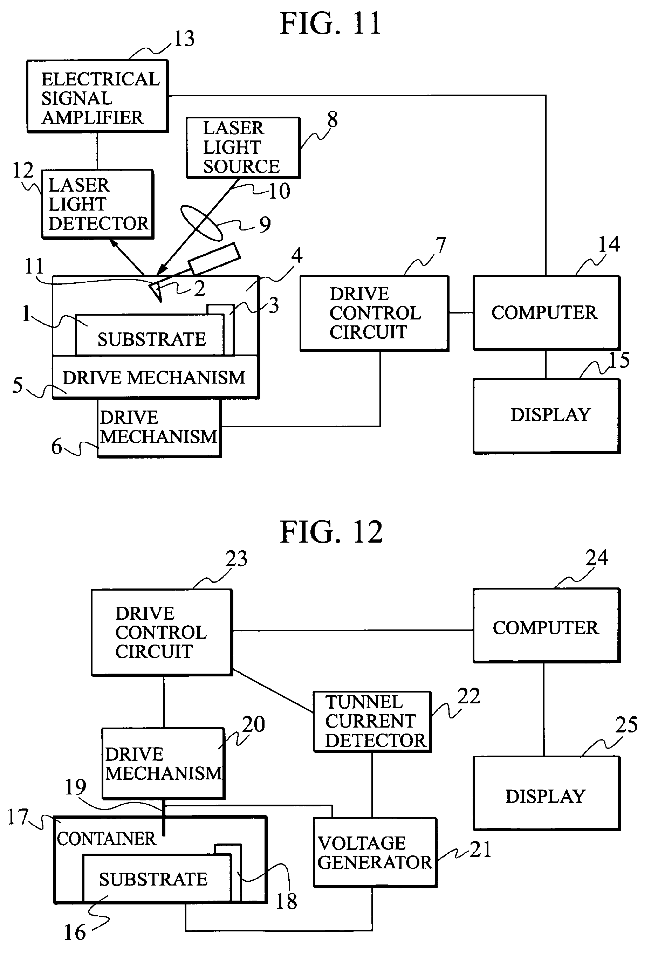

FIG. 11 is a diagram for explaining the principle of a molecular detection system employing AFM, which is one embodiment of the present invention.

A substrate 1, on which a chain molecule as a measurement target is immobilized, is set within a container 4 using a jig 3, and a probe 2 is housed in the container 4. Drive mechanisms 5 and 6 are arranged so that the substrate 1 can move in three dimensions, that is, in X axis, Y axis, and Z axis directions. A drive control circuit 7 sets a range in which the drive mechanisms 5 and 6 move, and the surface of the substrate 1 having the chain molecule immobilized thereon is scanned by the probe 2 while maintaining the probe 2 in proximity to or in contact with the surface of the sample.

Laser light 10 emitted from a laser light source 8 is focused by a lens 9 and falls on a probe back portion 11, and by capturing reflected light by means of a laser light detector 12, positional information regarding the probe 2 can be obtained. That is, when a repulsive force or an attractive force acts between the probe 2 and the substrate 1, the probe back portion 11 flexes accordingly. When the probe back portion 11 flexes, the angle at which the laser light 10 is reflected changes, and an electrical signal outputted from the laser light detector 12 changes accordingly. This electrical signal is transmitted to a computer 14 via an electrical signal amplifier 13. The drive control circuit 7 controls movement of the drive mechanisms 5 and 6 so that a constant electrical signal can always be obtained via the computer 14, that is, the atomic force acting between the probe 2 and the substrate 1 is always constant. By analyzing the positions in the X axis, Y axis, and Z axis directions of the drive mechanisms 5 and 6 using the computer 14, the profile of the chain molecule can be detected. This positional information may be read out by a self-detection method in which a change in flexural resistance is read out electrically.

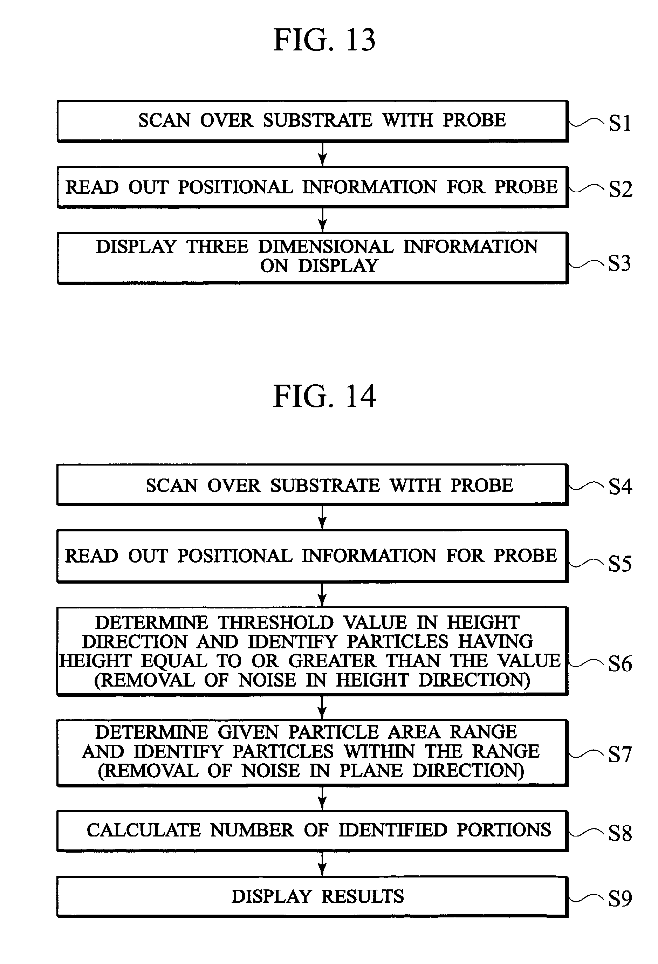

The molecular detection system of the present invention preferably has a device which visualizes the substrate having a chain molecule immobilized thereon. FIG. 13 is a flowchart showing the operation of the molecular detection system having the visualization device of the present invention. Firstly in S1 of FIG. 13, the probe 2 scans over the substrate 1. Subsequently, in S2 the detector 12 reads out positional information for the probe 2. Furthermore, in S3 the visualization device (computer 14) analyzes positional information on the X axis, the Y axis, and the Z axis of the drive mechanism 5 or 6, thereby obtains three dimensional information for the substrate 1 having the chain molecule immobilized thereon, that is, the profile of the chain molecule, and displays it on a display. By so doing, visualization information for the chain molecules per unit area on the substrate 1 can be obtained.

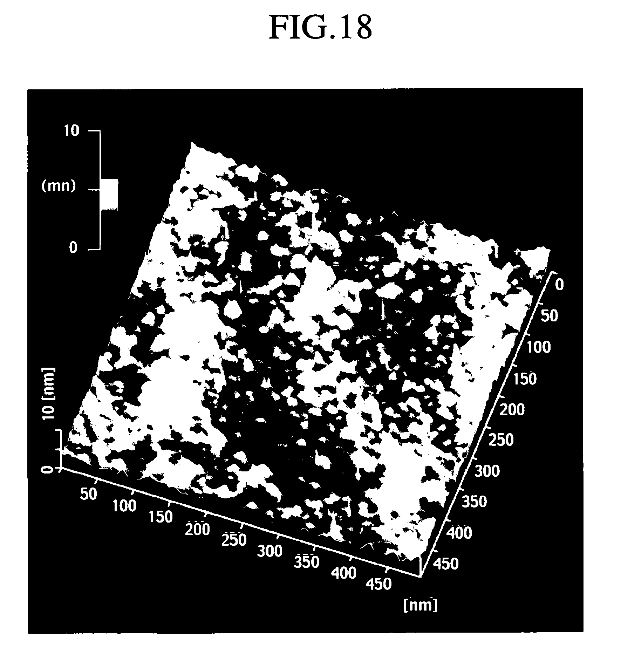

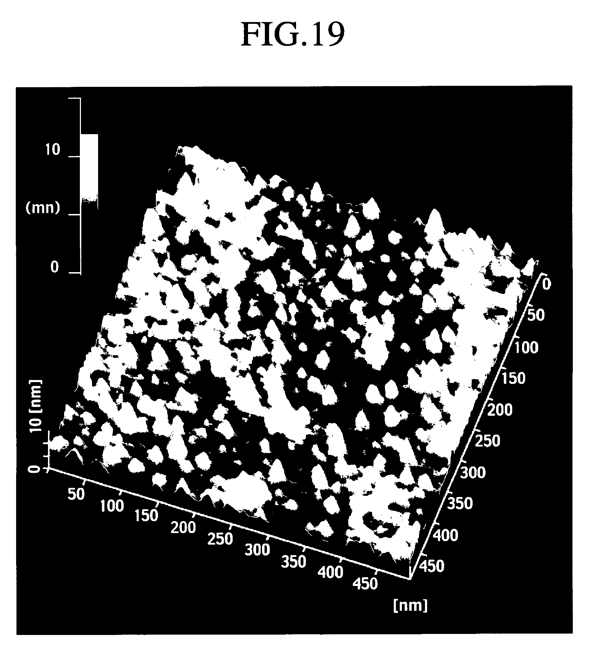

FIG. 18 and 19 are visualized substrates using the molecular detection system of the present invention. Although it depends on the visualization magnification, in FIG. 18 and 19 the chain molecules immobilized on the substrates are observed as particles.

The molecular detection system of the present invention preferably has a device which counts chain molecules. FIG. 14 is a flowchart showing the operation of the molecular detection system having the counting device of the present invention. In S4 of FIG. 14 the probe 2 scans over the substrate 1. In S5, the detector 12 reads out positional information for the probe 2. In S6, the counting device (computer 14) analyzes positional information for the X axis, the Y axis, and the Z axis of the drive mechanism 5 or 6 and thus recognizes particles (chain molecules) having a height greater than a given height (removal of noise in the height direction), and in S7 the counting device analyzes positional information for the X axis, the Y axis, and the Z axis of the drive mechanism 5 or 6 and thus recognizes particles having a given area (removal of noise in the plane direction). In S8, the counting device counts the number of particles recognized, and in S9 the results are displayed on a display. By so doing, the number of chain molecules probed per unit area on the substrate 1 can be counted.

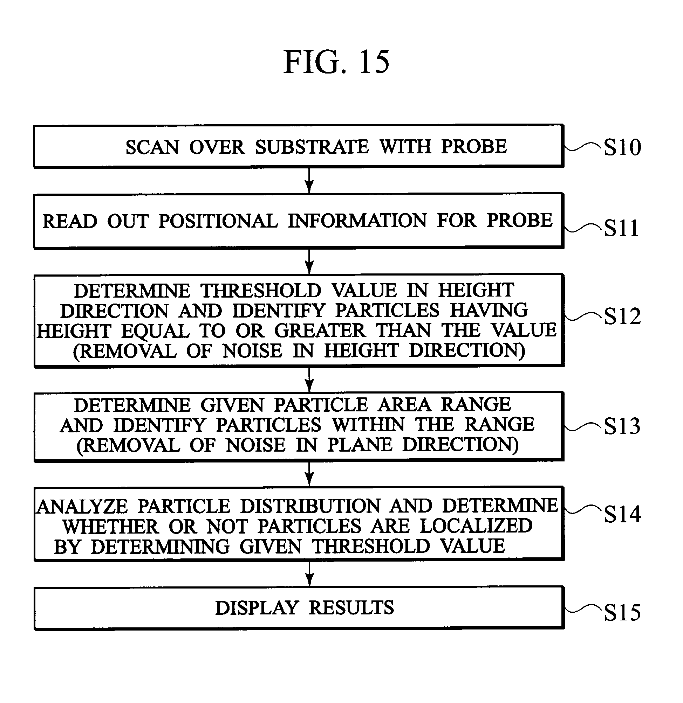

Furthermore, the molecular detection system of the present invention preferably has a device which provides information about the localization of chain molecules. FIG. 15 is a flowchart showing the operation of the molecular detection system having the localization information providing device of the present invention. In S10 of FIG. 15, the probe 2 scans over the substrate 1. In S11, the detector 12 reads out positional information for the probe 2. In S12, the localization information providing device (computer 14) analyzes positional information for the X axis, the Y axis, and the Z axis of the drive mechanism 5 or 6 and thus recognizes particles having a height less than a given height (removal of noise in the height direction), and in S13, the localization information providing device analyzes positional information for the X axis, the Y axis, and the Z axis of the drive mechanism 5 or 6 and thus recognizes particles having a given area (removal of noise in the plane direction). In S14, the localization information providing device analyzes the distribution of the recognized particles, and determines whether or not the particles are localized or nonlocalized in accordance with a given reference. In S15 the results are displayed on a display. By so doing, information about the localization of chain molecules per unit area can be obtained.

In S14, the particle distribution can be analyzed by a conventionally known method such as variance analysis or fractal analysis, by a method in which the distribution of distance between recognized particles is examined, or by a method in which an observed area on the substrate is divided into sections and the number of particles present in each section is compared and examined.

According to the intended application of the molecular detection system of the present invention, in S6 or S12, particles having a height less than a given height may be recognized, or particles having a given height may be recognized. In the same manner, in S7 or S13, particles having an area less than a given area may be recognized, or particles having an area equal to or greater than a given area may be recognized.

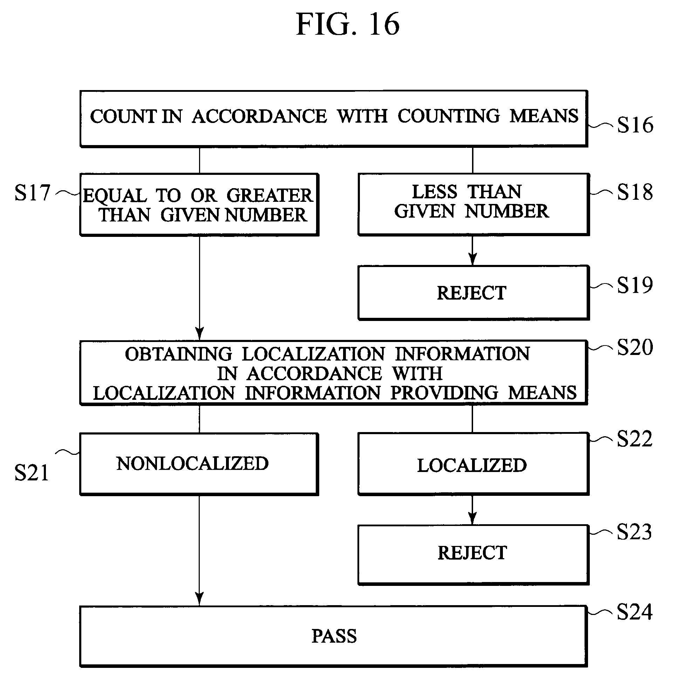

Moreover, the molecular detection system of the present invention may have a device which discriminates between substrates having chain molecules immobilized thereon based on information about visualization, counting, localization, etc. of chain molecules provided by the above-mentioned device. FIG. 16 is a flowchart showing the operation of device (computer 14) for discriminating between substrates having chain molecules immobilized thereon of the present invention. In S16 of FIG. 16, the numbers of chain molecules obtained by the counting device are discriminated in accordance with a given discrimination reference. When the number of chain molecules is equal to or greater than the given discrimination reference (S17), subsequently in S20 it is determined whether or not the chain molecules are localized or nonlocalized based on information about localization of the chain molecules obtained by the localization information providing device. When the number of chain molecules is less than the given discrimination reference (S18), the substrate 1 having the chain molecules immobilized thereon is rejected (S19). After discriminating between localization and nonlocalization in S20, if the chain molecules are nonlocalized (S21), the substrate 1 having the chain molecules immobilized thereon is passed (S24). If the chain molecules are localized (S22), the substrate 1 having the chain molecules immobilized thereon is rejected. By so doing, it is possible to inspect substrates having chain molecules immobilized thereon in a production process, and discriminate those that do not have a certain reference level. These results may be displayed on a display.

According to the intended application of the molecular detection system of the present invention, in S17 when the number of chain molecules is less than a given discrimination reference it may be passed, and in S18 when the number of chain molecules is equal to or greater than the given discrimination reference it may be rejected. Similarly, in S21 when the chain molecules are nonlocalized it may be rejected, and in S22 when the chain molecules are localized it may be passed.

It is also possible to inspect a substrate by employing, as the device which discriminates between substrates having chain molecules immobilized thereon, the obtaining of information about visualization, counting, localization, etc. of chain molecules for a plurality of sections of the substrate and examining the information thus obtained. For example, by discrimination between distributions of the results of counting chain molecules at 10 locations on the substrate, it is possible to discriminate between substrates that pass and those that are rejected.

As the container used in the molecular detection system of the present invention, any material such as resin, metal, or glass, and any shape may be used while taking into consideration the sample form and reactivity with the solution. The chain molecules immobilized on the substrate move around freely in solution, but in air adjacent molecules are intertwined with each other and stick to the substrate, and the molecules cannot be individually identified. Furthermore, when the molecules stick to the substrate, they lose activity. By use of the molecular detection system of the present invention, chain molecules on the substrate can be detected in solution. The solution used for measurement is not particularly limited as long as the chain molecules can exist stably, and it is preferable to use an aqueous solution containing a salt such as sodium chloride, potassium chloride, ammonium chloride, sodium acetate, potassium acetate, or ammonium acetate, or a buffer solution.

The probe 2 used in the molecular detection system of the present invention may employ any commercial product such as silicon, silicon oxide, silicon nitride, or carbon nanotube, but is not limited thereto. In the present invention it is preferable to use silicon nitride. The radius of curvature of the probe tip is preferably equal to or less than 20 nm. More preferably, it is equal to or less than 10 nm.

In the present invention, the drive mechanism 5 or 6 may be any of a piezoelectric element, a voice coil, and a mechanical mechanism, but is not limited thereto. The piezoelectric element is preferable.

In the present invention, any laser light source may be used as the laser light source 8. It is preferable to use a visible light semiconductor laser. Any device may be used as the laser light detector 12 as long as positional information can be captured two-dimensionally by light. It is preferably a photodiode, a CCD, or a CMOS device.

FIG. 12 is a diagram for explaining the principle of the molecular detection system employing STM, which is one embodiment of the present invention.

In FIG. 12, several elements shown in FIG. 11 are duplicated, and explanation of the elements common to both FIG. 11 and FIG. 12 is omitted. A voltage generator 21 is used for passing tunnel current between a probe 19 and a chain molecule on a substrate 16. Tunnel current is made to flow by making the probe 19 approach the chain molecule until it is a distance of a few atoms away by means of a drive mechanism 20. This current is detected by a tunnel current detection part 22 and converted into an electrical signal. The probe 19 is scanned using a drive control circuit 23 so as to give a constant tunnel current, and positional information in X axis, Y axis, and Z axis directions of the drive mechanism 20 can be detected as a profile of the chain molecule via a computer 24.

In the present invention, the drive mechanism of the AFM is not limited to a method in which the substrate is driven, but a method in which the probe is driven is also possible. Furthermore, the STM is not limited to a method in which the probe is driven, but a method in which the substrate is driven is possible.

As described above, in accordance with the method of the present invention, chain molecules immobilized on a substrate can be clearly detected, which has conventionally been difficult. In particular, outstanding effects in which, by probing uprightly disposed single strand molecules by means of a scanning probe microscope, the molecules can be identified and information about localization can be obtained; or in which, by binding a complementary single strand molecule to this single strand molecule so as to form a rigid multiple strand molecule, a clearer molecular image can be obtained and accurate information about localization can be obtained, etc, can be recognized. Furthermore, in accordance with the method of the present invention, regardless of the type and the length (height) of chain molecules immobilized on a substrate, or whether or not the chain molecules immobilized on a substrate are of the same type or a plurality of types, various molecules can be detected and counted, and information about localization can be given.

Furthermore, in accordance with the molecular detection system of the present invention, with respect to a DNA chip, a DNA microarray, a microtiter plate or, analogous thereto, a substrate with a biological material immobilized thereon, it is possible to visualize chain molecules while maintaining the chain molecules in an active state, count the number of chain molecules per unit area, and obtain information about the localization of chain molecules. This enables the substrate with a chain molecule immobilized thereon to be. inspected easily based on a given reference, and in a process for producing a substrate with a chain molecule immobilized thereon, a simple technique for inspecting a substrate can be provided.

The molecular detection system of the present invention is used as a system for inspecting a substrate with a chain molecule immobilized thereon such as a DNA chip, a DNA microarray, or a microtiter plate, and may also be used in a detection method in which, after nucleic acid hybridization, antigen-antibody reaction, receptor assay, etc. is carried out, instead of using conventional absorbance measurement, fluorescence intensity measurement, etc., using the substrate with a chain molecule immobilized thereon, the nucleic acid or protein is detected. In accordance with use of the molecular detection system of the present invention, since molecules can be individually recognized, compared with the conventional detection method, detection is possible with a small amount of sample, a high sensitivity detection method can be provided and, furthermore, the size of the chip, array, etc. can be reduced.

### Examples

Examples of the present invention are explained below, but the present invention is not limited to the examples below.

### Example 1

### <Substrate used>

As a substrate, a plastic substrate (1.5 cm × 1.5 cm) into which carboxyl groups had been introduced was used.

### <Immobilization of dT₂₀>

Firstly, a 25 mM EDC (1-ethyl-3-(3-dimethylaminopropyl)carbodiimide-HCl) solution was prepared using a 0.1 M MES (2-(*N*-morpholino)ethanesulfonic acid) buffer solution whose pH had been adjusted to 6.0 using sodium hydroxide. Subsequently, by use of this solution and dT₂₀ (5'-NH₂-(CH₂)₆₋(thymidine 5'-monophosphate)₂₀), a solution (1.3 µM dT₂₀/EDC solution) was prepared; 100 to 150 µL of this solution was dropped on the substrate, and after a reaction by incubating at 60°C for 6 hours, the substrate was washed with pure water so as to remove excess solution.

### <Observation of single strand nucleic acid>

The substrate with the single strand DNA immobilized thereon was immersed in a TE buffer solution (Tris-EDTA buffer solution, 10 mM Tris, 1 mM EDTA, pH 7.8, containing 0.5 M sodium chloride), and undulations of the surface of the substrate with dT₂₀ immobilized thereon were imaged by means of an atomic force microscope. As the atomic force microscope, a model SPI3800N (Seiko Instruments, Inc.) was used, and observation was carried out in DFM mode by scanning a region of 500 nm 500 nm.

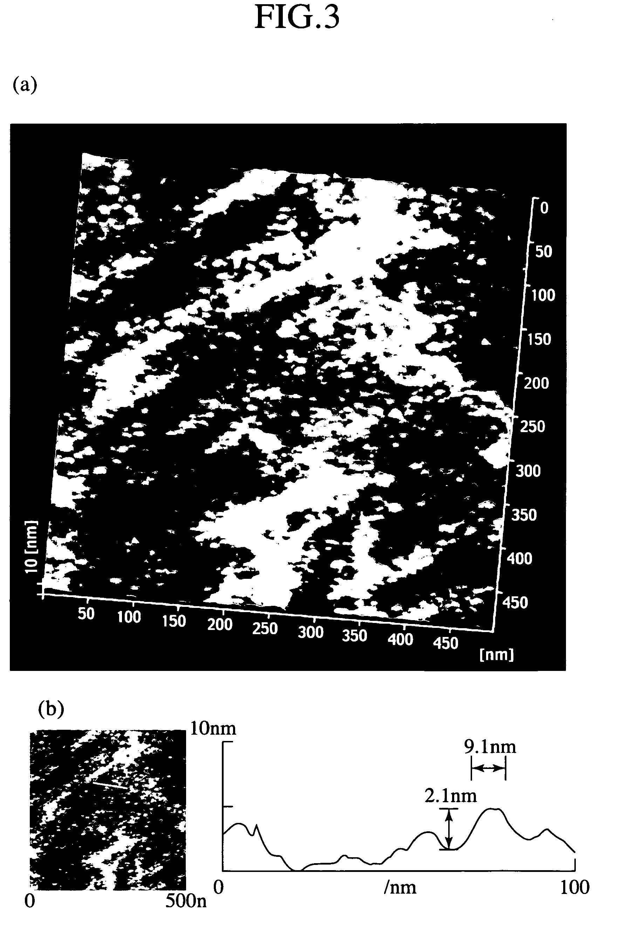

FIG. 3 is an image of the substrate observed with the atomic force microscope. Undulations were observed.

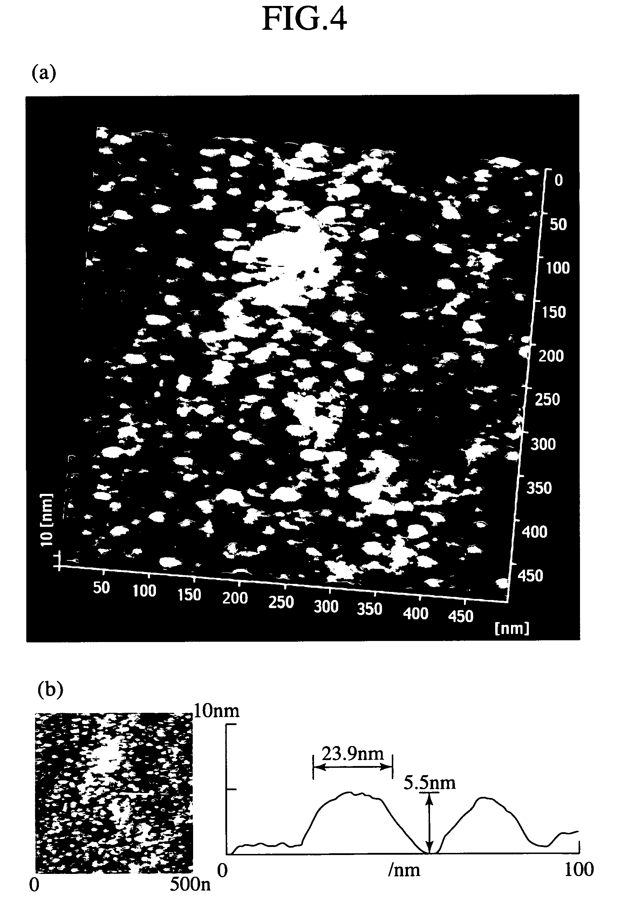

FIG. 4 is an image of the substrate with dT₂₀ immobilized thereon prepared by the method above observed with the atomic force microscope. Particles having a height of about 5 nm were observed.

### <Formation of double helix>

As a complementary single strand nucleic acid, dA₂₀-FAM (5'-5-carboxy-fluorescein-(CH₂)₆-(2'-deoxyadenosine 5'-monophosphate)₂₀) was used. This dA₂₀-FAM was dissolved in a TE buffer solution (20 pmol/µL). 100 to 150 µL of this solution was dropped on the substrate with dT₂₀ immobilized thereon. One hour later, excess dA₂₀-FAM solution was removed by washing with a TE buffer solution, the substrate was immersed in a TE buffer solution, and undulations of the surface of the substrate with dT₂₀ immobilized thereon were imaged by means of the atomic force microscope.

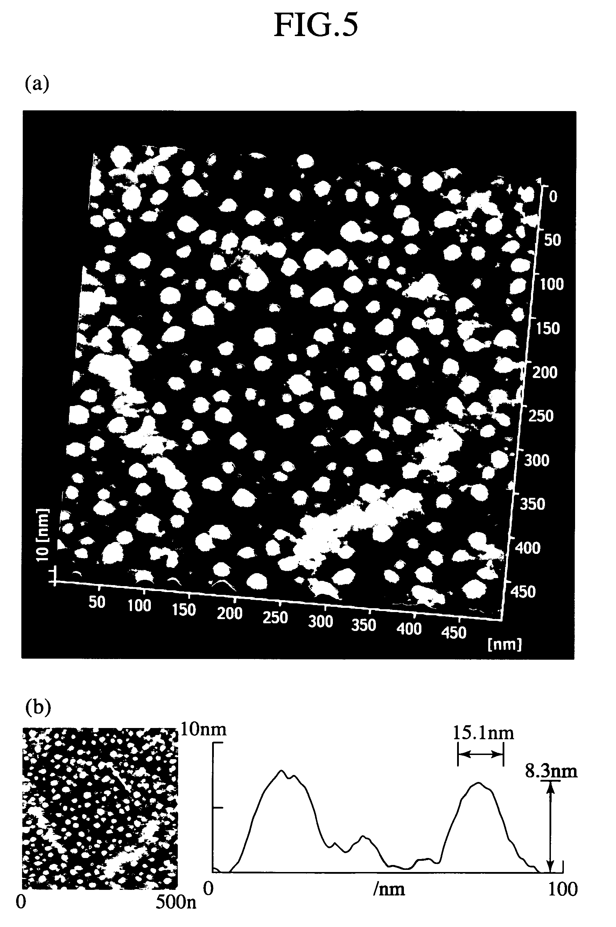

FIG. 5 is an image of the double strand nucleic acid formed by the above-mentioned method observed with the atomic force microscope. Particles having a height of 7 to 8 nm were clearly and regularly observed.

### <Image of double helix formed in air>

The surface of a substrate on which the immobilized dT₂₀ and dA₂₀-FAM formed a double helix was imaged in air by means of the atomic force microscope.

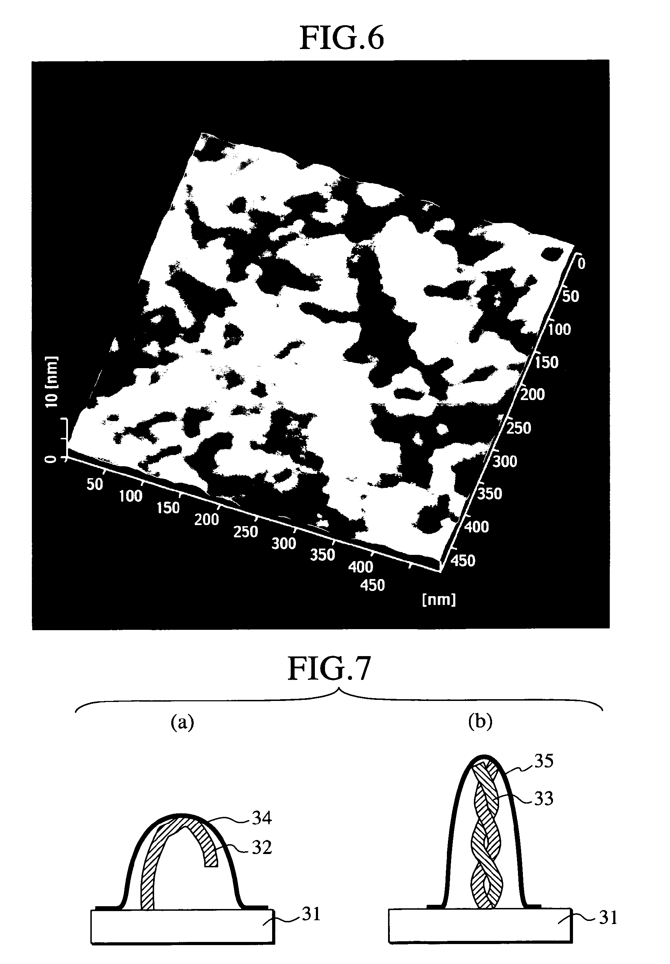

FIG. 6 is an image of the double strand nucleic acid formed by the above-mentioned method observed with the atomic force microscope in air instead of being immersed in a TE buffer solution. It can be confirmed that, unlike in FIG. 4, the height could not be discriminated.

FIG. 7 is a schematic diagram showing line profiles in FIG. 4 and FIG. 5. (a) shows a single strand DNA immobilized on the substrate, and (b) shows double strand DNA having identical numbers of bases immobilized on the substrate.

### Example 2

Particle analysis was carried out for the substrate used, the substrate with dT₂₀ immobilized thereon formed by the above-mentioned method, and the substrate on which a double helix of immobilized dT₂₀ and dA₂₀-FAM was formed by the above-mentioned method.

The particle analysis was carried out using software included with the model SPI3800N (Seiko Instruments, Inc.). The number was determined by setting a threshold value of 7.5 nm and excluding particles having a particle area of equal to or less than 50 nm².

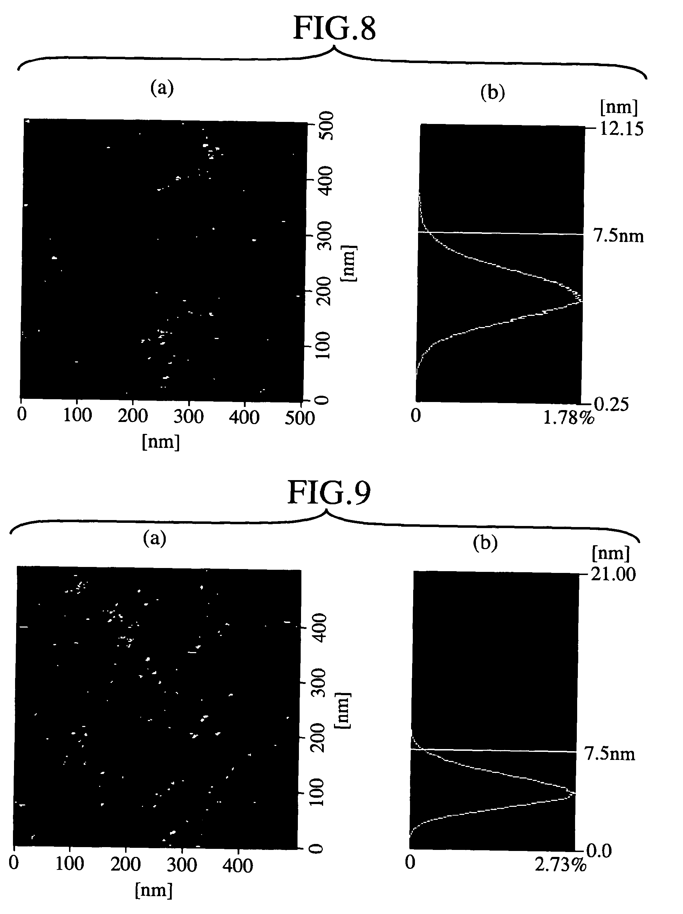

FIG. 8 is a particle analysis image of the substrate. Dark spots denote counted particles, and pale spots denote excluded particles. The number of particles having a height of equal to or greater than 7.5 nm was 14 in a region of 500 nm x 500 nm.

FIG. 9 is a particle analysis image of the substrate with dT₂₀ immobilized thereon. Dark spots denote counted particles, and pale spots denote excluded particles. The number of particles having a height of equal to or greater than 7.5 nm was 17 in a region of 500 nm x 500 nm.

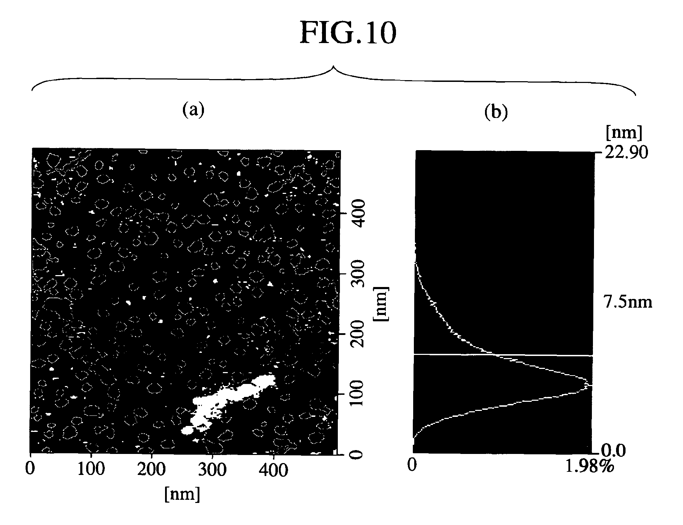

FIG. 10 is a particle analysis image of the substrate on which the double helix of immobilized dT₂₀ and dA₂₀-FAM was formed. Dark spots denote counted particles, and pale spots denote excluded particles. The number of particles having a height of equal to or greater than 7.5 nm was 250 in a region of 500 nm x 500 nm. It can be confirmed that the number of molecules can thus be obtained by counting.

### Example 3

### <Incorporation of functional group onto surface>

A polystyrene substrate (1.5 cm × 1.5 cm) was irradiated with a low pressure mercury lamp for 90 sec (500 mJ/cm²) so as to incorporate carboxyl groups.

### <Immobilization of dT₂₀ (single strand molecule)>

Firstly, a 25 mM EDC (1-ethyl-3-(3-dimethylaminopropyl)carbodiimide-HCl) solution was prepared using a 0.1 M MES (2-(N-morpholino)ethanesulfonic acid) buffer solution whose pH had been adjusted to 6.0 using sodium hydroxide. Subsequently, by use of this solution and dT₂₀ (5'-NH₂-(CH₂)₆₋(thymidine 5'-monophosphate)₂₀), a solution (1.3 µM dT₂₀/EDC solution) was prepared; 100 to 150 µL of this solution was dropped on the substrate, and after a reaction by incubating at 60°C for 6 hours, the substrate was washed with pure water so as to remove excess solution.

### <Observation of single strand nucleic acid (single strand molecule)>

The substrate with the single strand DNA immobilized thereon was immersed in a TE buffer solution (Tris-EDTA buffer solution, 10 mM Tris, 1 mM EDTA, pH 7.8, containing 0.5 M sodium chloride), and a region of 500 nm x 500 nm of the surface of the substrate with dT₂₀ immobilized thereon was scanned and visualized by means of the molecular detection system of the present invention.

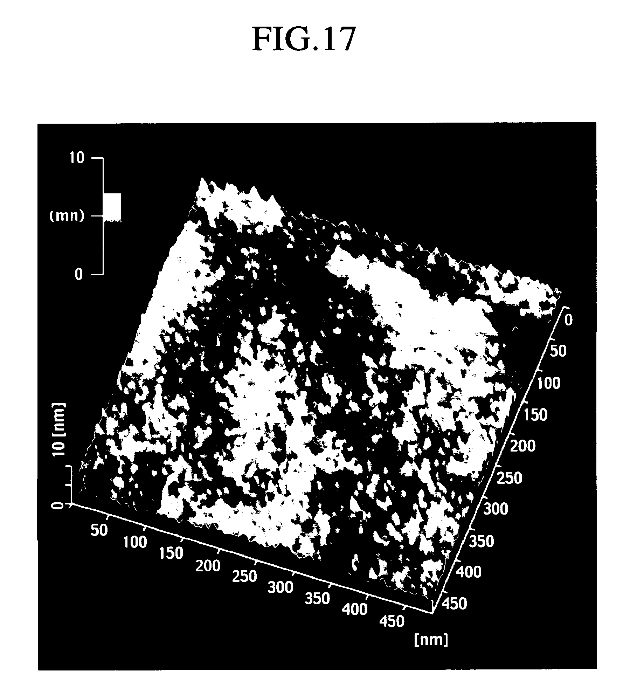

FIG. 17 shows the substrate visualized using the molecular detection system of the present invention in accordance with FIG. 13.

FIG. 18 shows the substrate with dT₂₀ immobilized thereon, formed by the above-mentioned method, visualized using the molecular detection system of the present invention in accordance with FIG. 13. It was confirmed that there were particles having a height of about 5 nm (chain molecules).

### <Formation of double helix (multiple strand molecule)>

As a complementary single strand nucleic acid, dA₂₀-FAM (5'-5-carboxy-fluorescein-(CH₂)₆-(2'-deoxyadenosine-5'-monophosphate)₂₀) was used. This dA₂₀-FAM was dissolved in a TE buffer solution (20 pmol/µL). 100 to 150 µL of this solution was dropped on the substrate with dT₂₀ immobilized thereon. One hour later, excess dA₂₀-FAM solution was removed by washing with the TE buffer solution.

### <Observation of double helix (multiple strand molecule)>

A region of 500 nm x 500 nm of the surface of the substrate with dT₂₀ immobilized thereon was scanned and visualized by means of the molecular detection system of the present invention in the above-mentioned TE buffer solution.

FIG. 19 shows the double strand nucleic acid, formed by the above-mentioned method, visualized using the molecular detection system of the present invention in accordance with FIG. 13. Particles having a height of about 8 nm (chain molecules) were observed clearly and regularly.

### Example 4

The substrate into which a functional group had been incorporated in Example 3, the substrate with dT₂₀ immobilized thereon, and the substrate on which the double helix was formed from dT₂₀ and dA₂₀-FAM were subjected to counting of molecules on the substrate in accordance with FIG. 14.

Since the height of the target molecules was about 8 nm, the threshold value in the height direction was set at 4.5 nm (molecules having a height of equal to or greater than 4.5 nm being detected) for the substrate with dT₂₀ immobilized thereon while taking into consideration bending of the molecules, and it was set at 7.5 nm (molecules having a height of equal to or greater than 7.5 nm being detected) for the functional group-incorporated substrate and the substrate on which the double helix of dT₂₀ and dA₂₀-FAM was formed. The particle area that was to be detected was set at equal to or greater than 50 nm² but no greater than 2,000 nm² for the cross sectional area of the particle at the threshold value in the height direction (molecules having a cross sectional particle area at the threshold value in the height direction of equal to or greater than 50 nm² but no greater than 2,000 nm² being detected).

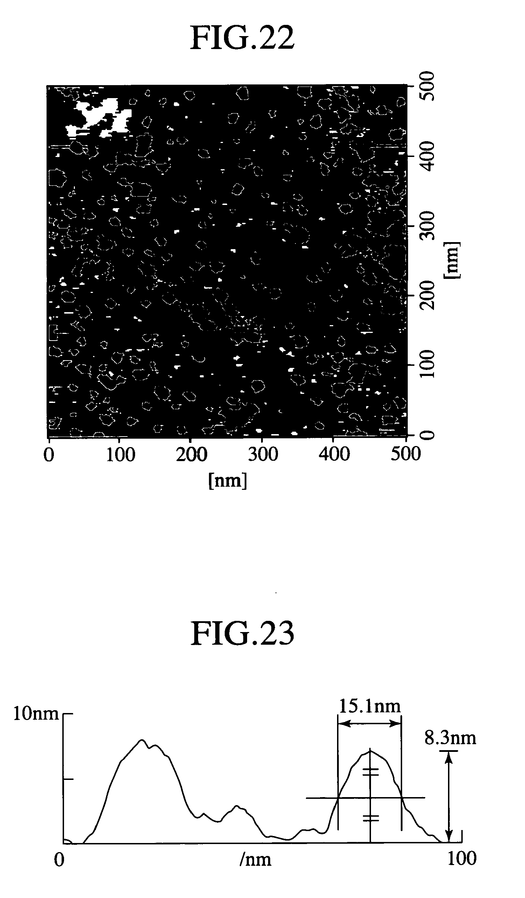

The range of the particle area set in this example was determined by the following method.
1) In S7 of FIG. 14, the counting device analyzes positional information for the X axis, the Y axis, and the Z axis of the drive mechanism 5 or 6 so as to give an image of the particle profile. Although it depends on the resolution, the single strand molecule of dT₂₀ and the multiple strand molecule of dT₂₀ and dA₂₀-FAM are recognized as a conical shape or a shape analogous to a mountain shape.
2) The topographic image of given particle is bisected at a sectional plane passing through the vertex thereof so as to give a line profile of the topographic image of the given particle (FIG. 23).
3) A half-width value of the line profile is determined, and an area of the cross section of the particle at a height of (particle height x 1/2) is determined from (diameter x 1/2)² x π, in which the half-width value obtained above is the diameter of the particle.
4) A value that is 30% of the area thus obtained is defined as a lower limit value of the range of particle area that is to be recognized (50 nm²).
5) A value that is 1000% of the area thus obtained is defined as an upper limit value of the range of particle area that is to be recognized (2000 nm²).

The method for determining the range of recognition area is not limited to the above-mentioned method, and an optimum method may be used according to the intended purpose for which the molecular detection system of the present invention is used.

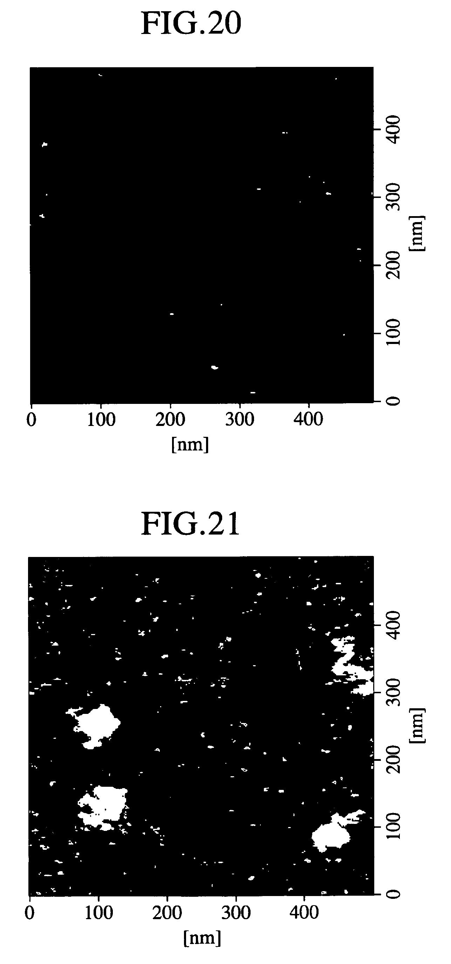

FIG. 20 is an analytical image of the substrate after noise had been removed. Black portions correspond to portions in which the height was less than 7.5 nm (portions removed as noise in the height direction), and pale spots correspond to portions in which the height was equal to or greater than 7.5 nm and the area was less than 50 nm² or greater than 2000 nm² (portions removed as noise in the plane direction). It was found from counting in a region of 500 nm x 500 nm that the number of chain molecules immobilized on the substrate was 0.

FIG. 21 is an analytical image of the substrate with dT₂₀ immobilized thereon after noise had been removed. Black portions correspond to portions in which the height was less than 4.5 nm (portions removed as noise in the height direction), pale spots correspond to portions in which the height was equal to or greater than 4.5 nm and the area was less than 50 nm² or greater than 2000 nm² (portions removed as noise in the plane direction), and dark spots correspond to portions in which the height was equal to or greater than 4.5 nm and the area was from 50 nm² to 2000 nm² (particles counted as chain molecules). It was found from counting in a region of 500 nm × 500 nm that the number of chain molecules immobilized on the substrate was 93.

FIG. 22 is an analytical image of the substrate on which the double helix of the immobilized dT₂₀ and dA₂₀-FAM was formed, after removing noise. Black portions correspond to portions in which the height was less than 7.5 nm (portions removed as noise in the height direction), pale spots correspond to portions in which the height was equal to or greater than 7.5 nm and the area was less than 50 nm² or greater than 2000 nm² (portions removed as noise in the plane direction), and dark spots correspond to portions in which the height was equal to or greater than 7.5 nm and the area was from 50 nm² to 2000 nm² (particles counted as chain molecules). It was found from counting in a region of 500 nm x 500 nm that the number of chain molecules immobilized on the substrate was 180.

### Example 5

As the substrate, a mica substrate (1.5 cm x 1.5 cm) was used.

### <Immobilization of lysozyme>

A 0.5 mg/mL lysozyme solution (SIGMA, EC 3.2.1.17) was prepared using a PBS buffer solution (containing 137 mM NaCl, 8.1 mM Na₂HPO₄, 2.7 mM KCl, and 1.5 mM KH₂PO₄). Subsequently, 50 to 100 µL of this solution was dropped on the substrate and incubated at room temperature for 30 min, and excess solution was then removed using the PBS buffer solution.

### <Observation of lysozyme>

The substrate with lysozyme immobilized thereon was immersed in the PBS buffer solution, and undulations of the surface of the substrate with lysozyme immobilized thereon were imaged by the atomic force microscope.

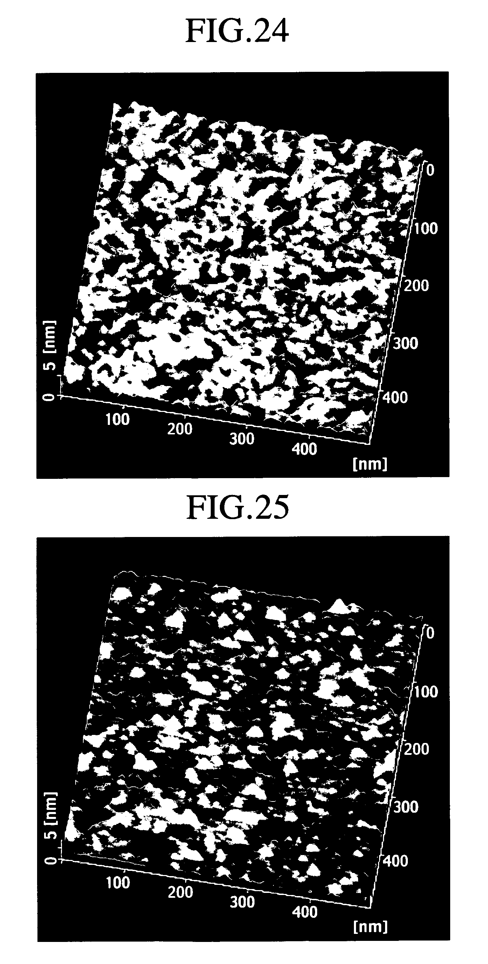

FIG. 24 is an image of the substrate with lysozyme immobilized thereon obtained by the above-mentioned method observed with the atomic force microscope. A large number of particles having a height of about 4 nm were observed.

Although it might be possible to observe lysozyme molecules individually in this state, it is difficult to discriminate active lysozyme, that is, lysozyme that can bind to an antibody. Therefore, reaction with an antibody for lysozyme was carried out.

### <Formation of antigen-antibody complex>

As the antibody, an anti-lysozyme antibody (ROCKLAND, IgG fraction of anti-Lysozyme [Hen Egg White] [Rabbit]) was used. This anti-lysozyme antibody was adjusted to 50 ng/µL using the PBS buffer solution. 100 µL of this solution was dropped on the mica substrate with lysozyme immobilized thereon and incubated for 2 hours, and excess solution was then removed using the PBS buffer solution. The substrate thus prepared was immersed in the PBS buffer solution, and undulations of the substrate were imaged using the atomic force microscope.

FIG. 25 is an image of the antigen-antibody complex formed by the above-mentioned method observed with the atomic force microscope. Particles having a height of about 12 nm were observed. The number of particles thus observed was equal to the number of lysozyme molecules that could bind to the antibody.

### Example 6

The substrate with lysozyme immobilized thereon, and the substrate on which an antigen-antibody complex between the immobilized lysozyme prepared by the above-mentioned method and the anti-lysozyme antibody was formed by the above-mentioned method were subjected to particle analysis.

The threshold value of the particle analysis was set at 4.5 nm, and the number of particles having a particle area from 50 to 200 nm² was counted.

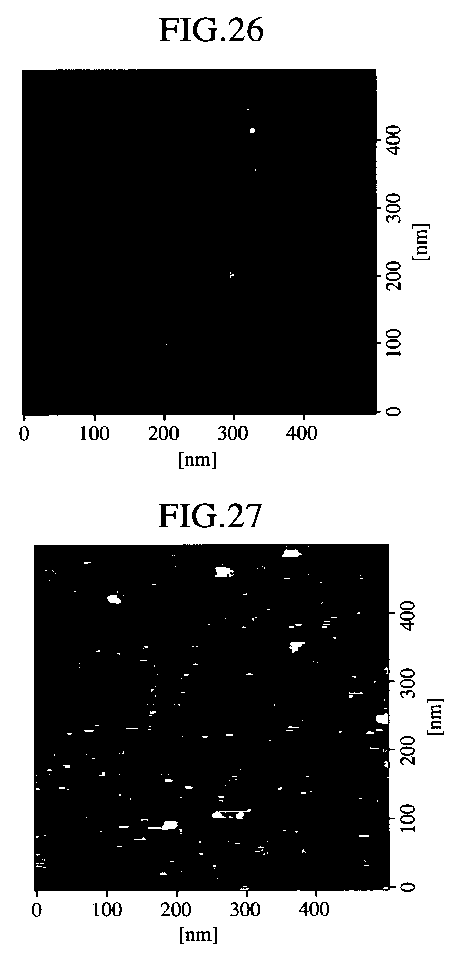

FIG. 26 is a particle analysis image of the substrate with lysozyme immobilized thereon. Dark spots correspond to counted particles, and pale spots correspond to excluded particles. The number of particles having a height of equal to or greater than 4.5 nm was 0 in a region of 500 nm x 500 nm.

FIG. 27 is a particle analysis image of the substrate on which the antigen-antibody complex was formed from the immobilized lysozyme and the anti-lysozyme antibody. Dark spots correspond to counted particles, and pale spots correspond to excluded particles. The number of particles having a height of equal to or greater than 4.5 nm was 51 in a region of 500 nm x 500 nm. The number of molecules could thus be obtained by counting.

## Claims

1. A molecular detection method comprising visualizing and identifying a chain molecule immobilized on a substrate by probing with a scanning probe microscope in solution.

2. The molecular detection method according to Claim 1, wherein the chain molecule immobilized on the substrate is an uprightly disposed single strand molecule.

3. The molecular detection method according to Claim 2, wherein the uprightly disposed single strand molecule is a nucleic acid, a peptide nucleic acid, a peptide, a glycopeptide, a protein, a glycoprotein, a polysaccharide, a synthetic polymer, or an analog thereof.

4. The molecular detection method according to Claim 1, wherein the chain molecule immobilized on the substrate is a multiple strand molecule comprising an uprightly disposed single strand molecule and at least one chain molecule that can bind to the single strand molecule.

5. The molecular detection method according to Claim 4, wherein the multiple strand molecule is a complex of one or more types of molecules selected from a nucleic acid, a peptide nucleic acid, a peptide, a glycopeptide, a protein, a glycoprotein, a polysaccharide, a synthetic polymer, or an analog thereof.

6. A molecular counting method comprising detecting a molecule by the method according to any one of Claims 1 to 5, and counting the number of detected chain molecules per unit area.

7. A molecular localization detection method comprising detecting a molecule by the method according to any one of Claims 1 to 5, and counting the number of detected chain molecules per unit area, thus giving molecular localization information.

8. A molecular detection system for detecting a chain molecule immobilized on a substrate, the system comprising a jig for holding the substrate, a container housing the substrate and a solution, a probe, a probe detector, a drive mechanism for scanning the substrate or the probe in three dimensions, and a drive control circuit for controlling the drive mechanism.

9. The molecular detection system according to Claim 8, wherein it further comprises a device which visualizes the chain molecule.

10. The molecular detection system according to either Claim 8 or 9, wherein it further comprises a device which counts the chain molecules.

11. The molecular detection system according to any one of Claims 8 to 10, wherein it further comprises a device which provides information about localization of the chain molecules.

12. The molecular detection system according to Claim 11, wherein it further comprises a device which discriminates between substrates with chain molecules immobilized thereon.

13. The molecular detection system according to any one of Claims 8 to 12, wherein the chain molecule immobilized on the substrate is a single strand molecule uprightly disposed on the substrate.

14. The molecular detection system according to Claim 13, wherein the uprightly disposed single strand molecule is a nucleic acid, a peptide nucleic acid, a peptide, a glycopeptide, a protein, a glycoprotein, a polysaccharide, a synthetic polymer, or an analog thereof.

15. The molecular detection system according to any one of Claims 8 to 12, wherein the chain molecule immobilized on the substrate is a multiple strand molecule comprising the uprightly disposed single strand molecule and at least one chain molecule that can bind to the single strand molecule.

16. The molecular detection system according to Claim 15, wherein the multiple strand molecule is a complex of one or more types of molecules selected from a nucleic acid, a peptide nucleic acid, a peptide, a glycopeptide, a protein, a glycoprotein, a polysaccharide, a synthetic polymer, or an analog thereof.

17. A production process for a substrate with a chain molecule immobilized thereon, the production process including the method according to any one of Claims 1 to 7.

18. A production process for a substrate with a chain molecule immobilized thereon, the production process employing the system according to any one of Claims 8 to 16.
